# EUROPEAN PATENT APPLICATION

(11) **EP 4 194 546 A1**
(43) Date of publication of application: **14.06.2023**
(21) Application number: 21213133.8
(22) Date of filing: 08.12.2021
(51) Int. Cl.: C12N 5/00, C12N 5/09

(54) **METHOD OF PRODUCTION OF ORGANOIDS AND USES THEREOF**

(71) Applicant: Université de Strasbourg, 67000 Strasbourg (FR); CENTRE NATIONAL DE LA RECHERCHE SCIENTIFIQUE (CNRS), 75016 Paris (FR)
(72) Inventor: MULLER, Christian, 67000 STRASBOURG (FR); EMHEMMED, Fathi, 67400 ILLKIRCH (FR)
(74) Representative: Plasseraud IP

(57) **Abstract**

The present invention provides a method for the preparation of organoids that allow the 3D culture or co-culture of cells in an environment that closely mimics the natural *in vivo* situation. The organoids are prepared from liquid pearls. They may be used in the variety of applications including drug testing and screening and personalized medicine.

## Description

### Background of the Invention

The majority of cell culture studies have been performed on 2-dimensional (2D) surfaces. Although these conventional 2D cell culture systems have tremendously improved our understanding of basic cell biology, they have proved to be insufficient and unsuitable for new challenges in cell biology as well as for pharmaceutical assays and drug screening (Aggarwal et al., Biochemical Pharmacology, 2009, 78(9):1083-1094; Hait, Nat. Rev. Drug Discov., 2010, 9(4): 253-254). Indeed, 2D cell culture systems fall short of reproducing the complex and dynamic environments of the *in vivo* situation, which are known to affect cell morphology, growth rates, contact geometries, transport properties, and numerous other cellular functions.

In particular, 2D environment does not accurately mimic the 3D *in vivo* environment in which cancer cells reside. Limitations of 2D cell culture include the lack of cell-cell and cell-extracellular matrix (ECM) signalling that occurs in an *in vivo* environment where such signals are essential to cancer cell differentiation and proliferation and a range of cellular functions (Bissell et al., Curr. Opin. Cell Biol., 2003, 15: 753-762). 2D cell systems do not allow for areas of hypoxia, heterogeneous cell populations (including stroma cells), varying cell proliferation zones (quiescent vs. replicating), soluble signal gradients, and different nutrient and metabolic waste transport (Jain et al., PNAS, 2001, 28(26): 14748-14750). In addition, in 2D-cultured cells, individual drug targets may not be expressed or the level of cell signaling may not be equivalent to that found *in vivo* (Ghosh et al., J. Cell Physiol., 2005, 204(2): 522-531). As a result, the unnatural 2D environment may provide inaccurate data regarding the predicted response of cancer cells to chemotherapeutics (Gurski et al., Oncol. Issues, 2010, 25: 20-25). In fact, using a 2D cell model, attrition rates of drug candidates for cancer treatment were calculated to be approximately 95% (Kola et al., Nat. Rev. Drug Discov., 2004, 3(8): 711-715; de Boo et al., Lab Anim., 2005, 33(4): 369-377; Knight, Lab. Anim., 2007, 35(6): 641-659; Hutchinson and Kirk, Nat. Rev. Clin. Oncol., 2011, 8(4): 189-190), stemming from *in vitro* drug efficacy values that did not translate to the clinic, as well as unforeseen toxicity issues. This results in the loss of hundreds of millions of dollars spent on pre-clinical and clinical trials.

Three-dimensional (3D) cell culture methods have been developed with the goal of producing the most *in* vivo-like structures possible; and their use in drug research and development has, therefore, been suggested as a potential link to bridge the gap between monolayer cultures and *in vivo* animal model studies (Mazzoleni et al., Genes Nutr. 2009, 4: 13-22; Yamada et al., Cell, 2007, 130: 601-610). In a 3D environment, cells tend to be more subjected to morphological and physiological changes in contrast to those grown in a 2D environment. 3D cell culture also grants the possibility to grow simultaneously two, or more than two, different cellular populations with co-cultures accurately reproducing cellular functions observed within a tissue. The 3D cell culture systems include methods that modify cell culture surfaces and therefore promote 3D culture formation by preventing cells from attaching to their surface; the hanging drop method which supports cellular growth in suspension; rotary systems that encourage cells to adhere to each other to form 3D spheroids; 3D scaffolds and matrices, which provide extracellular support and allow 3D cell growth; and microfluidic systems that support 3D cell culture.

Whilst strongly preferable to conventional 2D cell cultures, 3D approaches have many limitations including time-consuming production, relatively high cost, poor reproducibility, and lack of compatibility with high-throughput screening instruments. Therefore, there still exists a need in the art for improved 3D cell culture systems, in particular systems that are screening-compatible, providing early physiological relevant efficacy and toxicity data in cancer drug discovery and that could eventually lead to cost-effective and patient-specific *in vitro* models for drug testing.

### Summary of the Invention

The present Inventors have developed a system for culturing and evaluating cell growth in hydrophobic structures in the form of pearls that cause cancer cells to naturally generate 3-dimensional organoids. The cancer cells are grown in a liquid pearl consisting of a drop of medium comprising methylcellulose and coated with particles of hydrophobic fumed silica. The fumed silica shell ensures optimal gas exchange between the interior liquid and the surrounding environment. The 3D-system helps reduce incubation volumes, which makes the resulting organoids suitable for high-throughput applications. Furthermore, co-culture of different cell phenotypes *(e.g.,* blood, endothelium, cancerous tissues) may be achieved by spontaneous fusion of juxtaposed liquid pearls. The new organoid obtained by fusion contains the different cultured and/or pretreated cell phenotypes, and is equivalent to an animal model of peritoneal injection of cancer cells into a "nude" mouse *(i.e.,* a mouse lacking an immune system). Thus, a 24-well plate comprising such co-culture organoids represents the equivalent of 24 mice, but occupies an area of only 100 cm² and is not associated with the high cost and ethical issues of animal experimentations.

Consequently, the present invention provides a method for generating an organoid, the method comprising steps of:
(1) producing a liquid pearl for 3D cell culture;
(2) introducing cells in suspension inside the liquid pearl to obtain an organoid; and
(3) culturing the organoid obtained,
   wherein, the liquid pearl for 3D cell culture is produced by a method comprising steps of:
   (a) coating a surface of a Petri dish with hydrophobic fumed silica;
   (b) pipetting a volume v of a solution of methylcellulose in a cell culture medium and placing it on the coated surface of the Petri dish; and
   (c) applying a gentle orbital shake to the Petri dish to form a liquid pearl.

In certain embodiments, the method used for producing the liquid pearl for 3D cell culture further comprises a step of:
(d) pipetting the liquid pearl formed in step (c) and placing it in a well of a U-bottom multiwell-plate.

In certain embodiments, the solution of methylcellulose in a cell culture medium comprises between 0.1 and 1.5% (w/v) of methylcellulose, in particular between 0.1 and 0.7% (w/v) of methylcellulose, preferably 0.3% (w/v) of methylcellulose, and/or the cell culture medium is Dulbecco's' Modified Eagles Medium (DMEM).

In certain embodiments, in step (b), the volume v of the solution of methylcellulose in a cell culture medium is comprised between 50 µL and 250 µL.

In certain embodiments, in the cell suspension of step (b), the cells are suspended at a concentration range of 1×10² cells/mL to 1×10⁹ cells/mL, in particular of 1×10³ cells/mL to 5×10⁸ cells/mL or of 1×10⁴ cells/mL to 1×10⁷ cells/mL or of 1×10⁵ cells/mL to 5×10⁶ cells/mL.

In certain embodiments, the cells in suspension are stem cells, progenitor cells or differentiated cells of human or mammal origin.

In certain embodiments, the cells in suspension are primary cells, secondary cells or immortalized cells originating from healthy or diseased biological tissue or fluid.

In certain embodiments, the cells in suspension are derived from a patient suffering from a disease, in particular a cancer patient.

In certain embodiments, in step (2) of the method, the volume of cell suspension introduced inside the liquid pearl is comprised between 2 µL and 30 µL.

In another aspect, the present invention provides a method for generating a heterotypic organoid, the method comprising steps of:
(1') generating a first organoid inside a first liquid pearl using a method described above or providing a first organoid inside a first liquid pearl generated using a method described above, wherein the first organoid comprises a first cell phenotype or cell composition;
(2') generating a second organoid inside a second liquid pearl using a method described above or providing a second organoid inside a second liquid pearl generated using a method described above, wherein the second organoid comprises a second cell phenotype or cell composition;
(3') placing the first liquid pearl in close proximity to the second liquid pearl to allow fusion of the first and second liquid pearls into a fusion liquid pearl comprising a heterotypic organoid,
wherein the first cell phenotype or cell composition and the second cell phenotype or cell composition are different.

In certain embodiments, the method further comprises:
(4') generating a third organoid inside a third liquid pearl using a method described above or providing a third organoid inside a third liquid pearl generated using a method described above, wherein the third organoid comprises a third cell phenotype or cell composition;
(5') placing the third liquid pearl in close proximity to the fusion liquid pearl comprising the heterotypic organoid obtained in step (3') to allow fusion of the third liquid pearl and the fusion liquid pearl into a larger fusion liquid pearl comprising a heterotypic organoid.

In certain embodiments, the method is used for generating a cancer-like organoid, cells of the first cell phenotype are cancer cells, and cells of the second cell phenotype are selected from the group consisting of epithelial cells, immune cells, endothelial cells, and fibroblasts.

In certain embodiments, the method is used for generating a cancer-like organoid, cells of the first cell phenotype are cancer cells, cells of the second cell phenotype are endothelial cells, in particular vascular endothelial cells, and cells of the third cell phenotype are immune cells, in particular natural killer cells.

In yet another aspect, the present invention provides a method of assessing the effect of an agent on a property of a cell in an organoid, the method comprising steps of:
(I) generating an organoid using a method described above or providing an organoid generated using a method described above, wherein the cell is in contact with the agent inside the organoid; and
(II) assessing the effect of the agent on the property of the cell in the organoid.

In certain embodiments, the property of the cell is selected from the group consisting of survival, growth, proliferation, differentiation, migration, morphology, signaling, metabolic activity, gene expression, cell-cell interaction, and any combination thereof.

In certain embodiments, the agent is a cell of a different cell phenotype or is a synthetic or natural compound or molecule.

In certain embodiments, the method is high throughput and/or automated.

These and other objects, advantages and features of the present invention will become apparent to those of ordinary skill in the art having read the following detailed description of the preferred embodiments.

### Brief Description of the Drawing

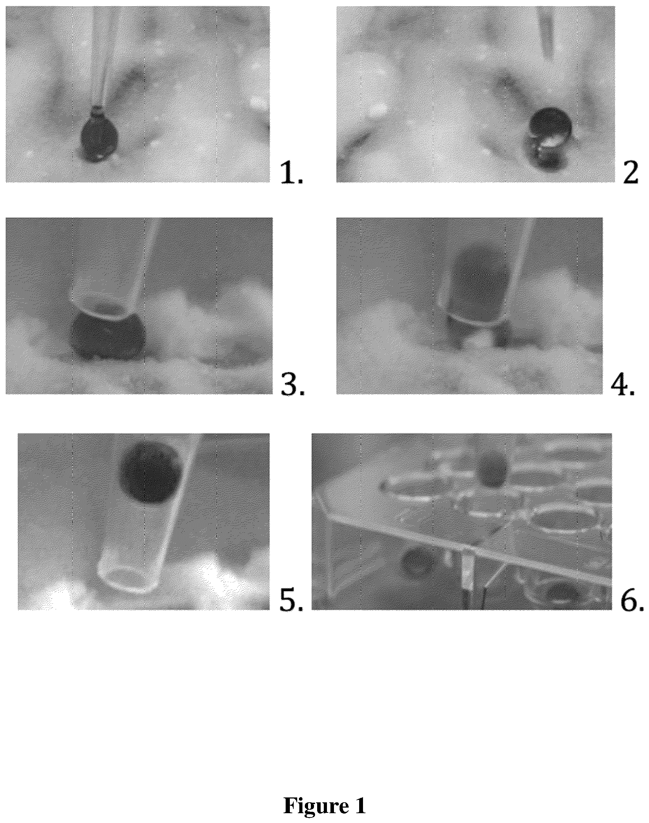

**Figure 1****.** Synopsis of obtaining beads in a Petri dish. (1) a 100 µL drop of medium containing (or not) cancerous cells in suspension is deposited on a thin layer of fumed silica; (2) gentle orbital agitation allows the drop to be coated with fumed silica; (3 to 5) the formed bead is pipetted with a cut cone; (6) finally the bead is deposited in a 96-well plate with round bottom wells.

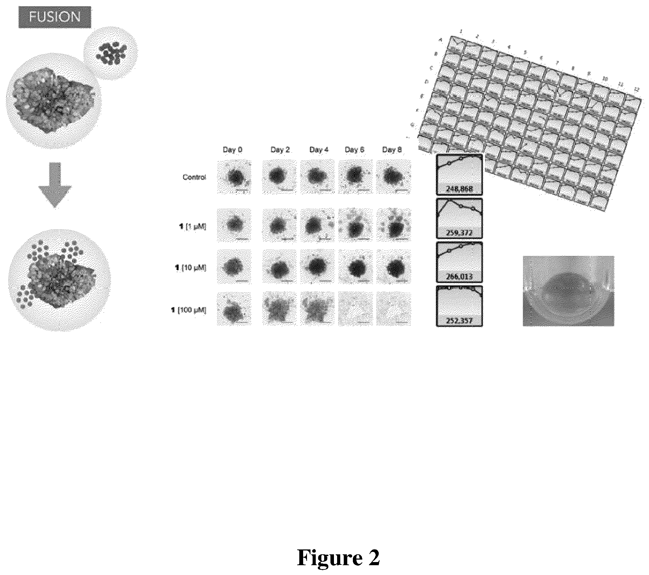

**Figure 2****.** Different examples of application. Left: an anti-cancer immuno-activation test: immune cells are activated in a pearl then the latter is fused with a pearl containing cancerous organoids. Middle: dose-effect curve of an anticancer agent (at 0; 1 µM; 10 µM, and 100 µM) as a function of time of culture (for 0; 2; 4; 6; and 8 days after the addition of the active molecule) in cartridge the growth curves of the organoid. Right: growth kinetics on a 96-well plate and photography of a pearl in a 96 well plate.

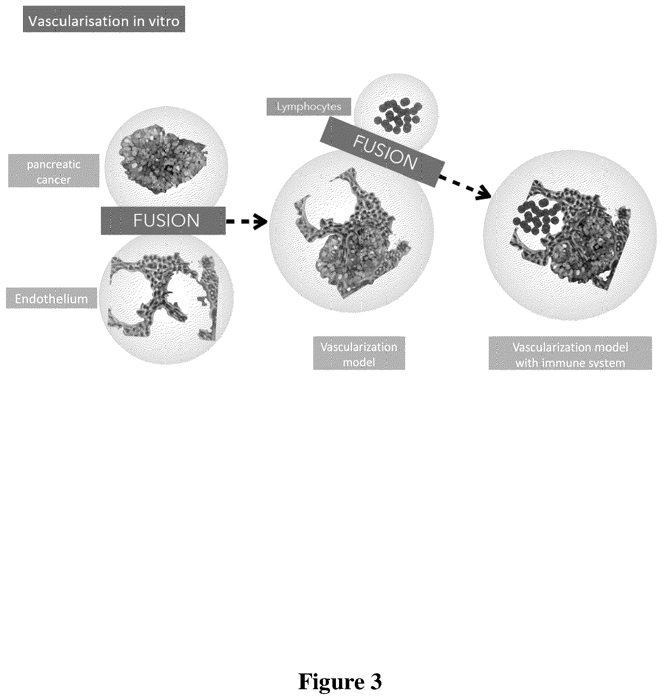

**Figure 3****.** Multi phenotypic organoid cultures closest to animal model. From Left to Right: cancerous organoids are produced in a pearl then fused with a pearl containing endothelial cells. Once fused, the co-culture can be fused to activated immune cells as described in Figure 2 in order to follow the fate of the cancerous organoid in the presence of epithelial cells.

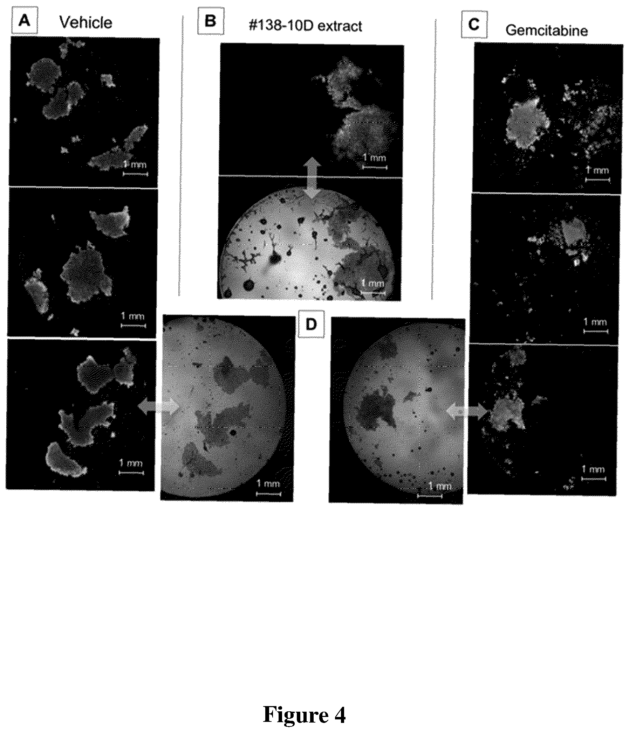

**Figure 4****.** Effects of an ethanolic plant extract on 3D cultured cells viability. Pancreatic cancer cells seeded at 10⁵ cells/mL were injected and grown in liquid pearls at a volume of 150 µL for 24 hours, then exposed to ethanolic extract #138-10D (80µg/ml), vehicle or Gemcitabine (80µM). After 24 hours of incubation, the cells were labeled (Calcein-AM = green-living cells and propidium iodide = red-dead cells). The composite image of the two channels was analyzed by imaging cytometry.

### Detailed Description of Certain Preferred Embodiments

As mentioned above, the present invention provides a method for generating organoids, in particular co-culture organoids, that more closely mimic the natural *in vivo* cell environment. The generation of organoids starts with the preparation of liquid pearls.

### I - Method of Preparation of Liquid Pearls

The terms ***"liquid marble"*** and ***"liquid pearl"*** are used herein interchangeably. They refer to a droplet of fluid encapsulated by a layer of hydrophobic powder. Liquid pearls are generally formed by rolling a small amount of liquid (in the order of tens or hundreds of microliters) over a bed or a layer of hydrophobic particles, which spontaneously cover the droplet surface. Due to the hydrophobicity of its shell, a liquid pearl can be rolled on a solid surface as a glass sphere while still being able to be deformed as a foam ball. In other words, liquid pearls exhibit reduced adhesion to solid surfaces, and movement without leakage of the internalized liquid.

A method for preparing a liquid pearl for 3D cell culture according to the present invention comprises steps of:
(a) coating a surface of a Petri dish with hydrophobic fumed silica;
(b) pipetting a volume v of a solution of methylcellulose in a cell culture medium and placing it on the coated surface of the Petri dish; and
(c) applying a gentle orbital shake to the Petri dish to form a liquid pearl.

In general, the method further comprises a step of:
(d) pipetting the liquid pearl formed in step (c) and placing it in a well of a U-bottom multiwell-plate.

### 1. Hydrophobic Fumed Silica and Petri Dish Coating

As used herein, the term ***"hydrophobic silica"*** refers to a form of silicon dioxide (commonly known as silica) that has hydrophobic groups chemically bonded to the surface. The hydrophobic groups are generally alkyl or polydimethylsiloxane chains. When hydrophobic silica is produced using a flame pyrolysis process, it is called ***"hydrophobic fumed silica"*** or ***"hydrophobic pyrogenic silica"*** and consists of microscopic droplets of amorphous hydrophobic silica fused into branched, chainlike, three-dimensional secondary particles which agglomerate into tertiary particles. Hydrophobic fumed silica is a powder with an extremely low bulk density and high surface area. It is used in cosmetics for its light-diffusing properties and as a light abrasive in products like toothpaste. Other uses include filler in silicone elastomer and viscosity adjustment in paints, coatings, printing inks, adhesives, and unsaturated polyester resins. Fumed silica is not listed as a carcinogen by EU-OSHA (European Agency for Safety and Health at Work), IARC (International Agency for Research on Cancer), or NTP (National Toxicology Program).

Any hydrophobic fumed silica may be used in the context of the present invention. Examples of fumed silica manufacturers include, but are not limited to, Evonik Industries (Germany), Cabot Corporation (US), Wacker Chemie AG (Germany), Tokuyama Corporation (Japan), Orisil (Ukraine), ICMD (Australia), Henan Xunyu Chemical (China) and Applied Material Solutions Inc. (US). Examples of hydrophobic fumed silica suitable for use in the context of the present invention include, but are not limited to, the products commercialized under the following trademarks: CAB-O-SIL^{®} TS-530 (of Cabot Corporation), REOLOSIL^{®} (of Tokuyama), AEROSIL^{®} 200 (of Evonik), HDK-N20^{®} (of ICMD Australia), ORISIL^{®} 200 (of Orisil), and XYSIL^{®} 200 (of Henan Xunyu Chemical).

The first step of the method for preparing a liquid pearl for 3D cell culture consists in coating a surface of a Petri dish with a hydrophobic fumed silica. As used herein, the term ***"Petri dish"*** has its art understood meaning and refers to a shallow transparent lidded dish that is generally used to hold growth medium in which cells can be cultured. Also called Petri plate or cell-culture dish, a Petri dish is the most common type of culture plate. Petri dishes are usually cylindrical, mostly with diameters ranging from 30 to 200 mm, and a height to diameter ratio ranging from 1:10 to 1:4. Squarish versions are also available. As will be understood by one skilled in the art, a different container with a flat surface may be used instead of a Petri dish.

Coating a surface of a Petri dish with hydrophobic fumed silica means covering at least a portion of the surface of a Petri dish with a layer of particles of hydrophobic fumed silica. As will be recognized by one skilled in the art, the amount of hydrophobic pyrogenic silica used to coat a Petri dish's surface will depend on the number of liquid pearls intended to be prepared. Furthermore, after preparation of a given number of liquid pearls, hydrophobic fumed silica may be resupplied to coat the surface of the Petri dish.

For example, the present Inventors have used a thin layer of around 1 to 2 mm of hydrophobic fumed silica in a Petri dish having a 6 cm-diameter to form up to 6 pearls at a time. Once the pearls are formed and removed, a second preparation of up to 6 pearls can be carried out in the same Petri dish. Before a third preparation is started, a new layer of hydrophobic fumed silica is preferably added to the Petri dish.

### 2. Solution of Methylcellulose in a Cell Culture Medium

In the context of the present invention, the liquid phase of the liquid pearls consists of a solution of methylcellulose in a cell culture medium. More specifically, the liquid phase comprises between 0.1 and 2% (w/v) of methylcellulose in a cell culture medium, in particular between 0.1 and 1.5% (w/v) of methylcellulose in a cell culture medium, for example between 0.1 and 0.7% (w/v), such as for example, 0.1% (w/v), 0.2% (w/v), 0.3% (w/v), 0.4% (w/v), 0.5% (w/v), 0.6% (w/v) or 0.7% (w/v). In certain preferred embodiments, the liquid phase used in the preparation of a liquid pearl comprises about 0.3% (w/v) of methylcellulose in a cell culture medium.

The terms ***"methylcellulose"*** and ***"methyl cellulose"*** are used herein interchangeably, and refer to a partial methyl ether of cellulose (CAS number: 9004-67-5) obtained by treating vegetable fibers with alkali and methyl chloride. Methylcellulose is a food additive under code E461 that is employed as an emulsifier, foaming agent, thickener and/or gelling agent. The European legislation has identified methylcellulose as a "quantum satis" food additive (no maximum authorized dose). As such, it has undergone all toxicological evaluations demonstrating its safety in the context of food use. Methylcellulose is also used in human medicine to treat constipation as a bulk forming laxative and in the manufacture of ophthalmic solutions for the treatment, control, prevention, and improvement of diseases, conditions, and symptoms of dry eyes. Methylcellulose is also used in the manufacture of drug capsules; its edible and nontoxic properties provide a vegetarian alternative to the use of gelatin. Methylcellulose is soluble in cell culture medium. In the practice of the present invention, the purpose of its use in forming liquid pearls is to enhance the viscosity of the cell culture medium. It provides cells to be cultured in a liquid pearl with a semisolid matrix that best mimics extracellular structures *in vivo* and helps keep the liquid pearl intact.

The terms ***"cell culture medium"*** and ***"basal medium"*** are used herein interchangeably. They refer to a culture medium that contains nutrients (inorganic salts, sugars, amino acids, and optionally, vitamins, organic acids and/or buffers or other well known cell culture nutrients) necessary to support the growth and/or survival of cultured cells, including co-cultured cells. A cell culture medium may or may not further comprise growth factors. A wide variety of commercially available basal media are well known to those skilled in the art, and include Dulbeccos' Modified Eagles Medium (DMEM), Roswell Park Memorial Institute Medium (RPMI), Iscove modified Dulbecco's' medium and Ham's medium. In certain preferred embodiments, the cell culture medium is Dulbecco modified Eagle's minimal essential medium (DMEM), which is a widely used basal medium for supporting the growth of many different mammalian cells. As known in the art, DMEM comprises inorganic salts, essential and non-essential amino acids, vitamins, glucose, and sodium pyruvate. DMEM may be high-glucose type (containing 4500 mg/L glucose) or low-glucose type (containing 1000 mg/L glucose). The low-glucose type DMEM is simply called DMEM.

A solution of methylcellulose in a cell culture medium suitable for generating liquid pearls may be prepared using any appropriate method known in the art. In certain embodiments, the solution of methylcellulose in a cell culture medium is prepared from a methylcellulose stock solution.

### A. Methylcellulose Stock Solution

A methylcellulose stock solution is a solution of methylcellulose in a cell culture medium, wherein, methylcellulose is present at a concentration comprised between 0.5 and 2.5% w/v, preferably between 1.0 and 2% w/v, for example about 1.0% w/v, about 1.1% w/v, about 1.2% w/v, about 1.3% w/v, about 1.4% w/v, about 1.5% w/v, about 1.6% w/v, about 1.7% w/v, about 1.8% w/v, about 1.9% w/v or about 2.0% w/v. More preferably, in a stock solution according to the present invention, methylcellulose is present at a concentration of about 1.5% w/v.

The stock solution of methylcellulose may be prepared using any appropriate method. For example, a stock solution of methylcellulose at a concentration of 1.5% w/v may be prepared using a method comprising steps of:
(i) heating a heating a volume V of DMEM in a water bath at 60°C for 20 minutes;
(ii) adding a mass m of methylcellulose to the heated DMEM such that the concentration of methylcellulose in the resulting mixture is 3% w/v and magnetically stirring the resulting mixture until dissolution of the methylcellulose;
(iii) adding a volume V of cold high-glucose DMEM supplemented with 20% fetal bovine serum and 1% Penicillin and Streptomycin;
(iv) magnetically stirring the mixture obtained in step (iii) overnight at 4°C in order to obtain a homogeneous solution;
(v) centrifuging the homogeneous solution at room temperature; and
(vi) recovering the supernatant.

In step (ii), dissolution of the methylcellulose is generally achieved after 5 to 10 minutes of stirring.

In step (iii), cold high-glucose DMEM corresponds to high-glucose DMEM stored at about 4°C.

The centrifugation step (step (v)) may be performed at 2000 g for 2 hours at room temperature.

The volume V will generally be comprised between 10 and 200 mL. For example, the volume V may be 50 mL, 75 mL, or 100 mL. However, it is to be understood that the method may be scaled-up or scaled-down depending on the needs. When the volume V of DMEM in step (ii) is 50 mL and the volume V of high-glucose DMEM in step (iii) is 50 mL, the mass m of methylcellulose is 1.5 g.

The recovered supernatant may be aliquoted and stored at 4°C. Under these storage conditions, the methylcellulose stock solution may be used for up to a year.

It will be recognized by one skilled in the art that variations of the above-described method may be conceived and developed without departing from the spirit of said method.

### B. Solution of Methylcellulose in a Cell Culture Medium

The solution used for producing liquid pearls according to the present invention is prepared by diluting the methylcellulose stock solution with a cell culture medium, preferably with high-glucose DMEM. Depending on the concentration of the methylcellulose stock solution, one skilled in the art knows how to calculate the appropriate dilution ratio. For example, using a methylcellulose stock solution at a concentration of 1.5 % w/v, one can dilute 20 mL of the stock solution with 80 mL of high-glucose DMEM to obtain a solution of methylcellulose at a concentration of 0.3% w/v.

A solution of methylcellulose in a cell culture medium described herein is generally used within one or several days (for example 2, 3, 4 or 5 days) after preparation and is stored at 4°C.

### 3. Preparation of Liquid Pearls

In step (b) of the method of preparation of liquid pearls for 3D cell culture, a volume v of the solution of methylcellulose in a cell culture medium is pipetted and placed on the surface of a Petri dish coated with hydrophobic fumed silica. The volume v is generally in the tens or hundreds of microliters. In certain preferred embodiments, the volume v is comprised between 50 µL and 250 µL, for example about 100 µL, about 150 µL, about 200 µL, or about 250 µL.

After the droplet of solution of methylcellulose in a cell culture medium is placed on the coated Petri dish, a gentle orbital shake is applied to the Petri dish (step (c) of the method). The term ***"orbital shake"*** refers to a circular shaking motion, that does not create vibrations. A ***"gentle orbital shake"*** refers to a circular shaking motion with a slow speed ranging from about 25 rpm to about 500 rpm, for example, for example from about 50 rpm to about 250 rpm, in particular for about 50 to about 100. Preferably, the gentle orbital shake applied to the Petri dish has a speed of 75 rpm. As known in the art, an orbital shake may be applied to the Petri dish using an orbital shaker device. An orbital shaker device is a mixing or stirring device used especially in scientific applications for mixing or stirring containers on a platform. Specifically, an orbital shaker device translates a platform in a manner such that all points on the upper surface, in the x-y plane, of the platform move in a circular path having a common radius. Orbital laboratory shakers are commercially available for example from Sigma-Aldrich, Thermo Fisher Scientific, VWR, Cole-Parmer, and the like.

The orbital shaking motion induces the formation of a liquid pearl by causing the liquid droplet to roll on the particles of hydrophobic fumed silica, which spontaneously cover the surface of the liquid droplet. The orbital shaking is performed for the time necessary for a liquid pearl to be formed, generally for a duration of between 5 and 30 seconds, for example between 10 and 20 seconds.

Once the liquid pearl is formed, it is pipetted, preferably using a shortened cone, and placed in a well of a U-bottom multiwell plate. A U-bottomed well is a well with a round-shape bottom *(i.e.,* which, in contrast to a flat-bottom well, has no edges). The format of the multiwell plate may be chosen from the commonly used formats in particular microwell plates, such as 24-well microplates, 48-well microplates, 96-well microplates, and the like. In certain embodiments, a liquid pearl is placed in each well of a microplate.

### II - Organoids and 3D Cell Culture

Liquid pearls prepared as described above may be used in a variety of applications, in particular as miniature bioreactors to produce organoids, in particular cancer-like organoids. The terms **"*organoid*", "*3D organoid*",** and **"*multicellular organoid*"** are used herein interchangeably. They refer to an aggregate, cluster or assembly of cells cultured to allow 3D growth. An organoid is substantially spherical, is stable, and can be lifted or relocated without disintegration. An organoid may contain a single cell type (homotypic organoid) or it may contain more than one cell type (heterotypic organoid).

Thus, the present invention relates to a method for generating an organoid, the method comprising steps of:
(1) producing a liquid pearl for 3D cell culture using a method as described herein or providing a liquid pearl produced using a method as described herein;
(2) introducing cells in suspension inside the liquid pearl to obtain an organoid; and
(3) culturing the organoid obtained.

### 1. Obtaining an Organoid

### A. Cells

Cells that can be introduced inside a liquid pearl for 3D culture in an organoid include any cells capable of being maintained in culture.

Cells used in the context of the present invention are mammalian cells such as human cells or cells from animals (*e*.*g*., primate, mouse, rat, rabbit, dog, cat, horse, cow, goat, pig, and the like), including laboratory animals. In many preferred embodiments, cells are of human origin. Mammalian cells may originate from any organ, fluid, or tissue (*e.g.*, brain, liver, skin, lung, kidney, pancreas (including islet cells), heart, muscle (including cardiac muscle), bone, cartilage, tendon, bone marrow, blood vessels, cornea, blood, amniotic fluid, umbilical cord blood, bladder, prostate, esophagus, stomach, and the like). It is appreciated that the cells to be cultured in organoids may be derived from a normal or healthy biological tissue or fluid, or from a biological tissue or fluid afflicted with a disease or illness, such as, for example, from a fluid or tissue derived from a tumor (see below).

As will be recognized by one skilled in the art, the cells to be cultured in organoids are isolated cells. The term **"*isolated cell*"** or **"*isolated population of cells*"** means any cell or population of cells that does not naturally occur in nature, *i.e.*, any cell or population of cells that is removed from its natural environment and/or is selected, modified, transformed (*e.g.*, genetically engineered), grown, or manipulated in any way, for example for the production of a substance by the cell *(e.g.,* a gene or protein of interest).

The term **"*cells*",** as used herein, is intended to include stem cells, progenitor cells, and differentiated cells.

As used herein, the term **"*stem cells*"** refers to relatively undifferentiated cells that have the capacity for sustained self-renewal as well as the potential to give rise to differentiated progeny *(i.e.,* to different types of specialized cells). Examples of stem cells that can be grown in an organoid according to the present invention include, but are not limited to, embryonic stem cells, adult stem cells, and induced pluripotent stem cells. The term **"*embryonic stem cells*"** refers to stem cells derived from a group of cells called the inner cell mass, which is part of the early (4 to 5 days old) embryo called the blastocyst. "Human embryonic stem cells" are embryonic stem cells of human origin that are generally derived from fertilized embryos that are less than one week old. *In vitro,* embryonic stem cells can proliferate indefinitely, a property that is not shared by adult stem cells. The terms **"*adult stem cells*"** and **"*somatic stem cells*"** are used herein interchangeably. They refer to undifferentiated cells, found throughout the body after development, that multiply by cell division to replenish dying cells and regenerate damaged tissues. They range from cells that are able to form many different kinds of tissues to more specialized cells that form just some of the cells of a particular tissue or organ. Unlike embryonic stem cells, somatic stem cells are found in juvenile, adult animals, and humans. The term **"*induced pluripotent stem cells*"** refers to a type of pluripotent stem cells artificially derived from non-pluripotent cells *(e.g.,* adult somatic cells). Induced pluripotent stem cells are identical to embryonic stem cells in their ability to form any differentiated cells, but are not derived from an embryo. The terms **"*human induced pluripotent stem cells*"** and **"*human iPS cells*"** are used herein interchangeably. They refer to induced pluripotent stem cells of human origin. Typically, a human induced pluripotent stem cell may be obtained through the induced expression of Oct3/4, Sox2, Klf4, and c-Myc genes in any adult somatic cell (*e.g.*, fibroblast).

The terms **"*progenitor cells*"** and **"*precursor cells*"** are used herein interchangeably. They refer to cells that occur in fetal or adult tissue and are partially specialized. These cells divide and give rise to differentiated cells. Progenitor or precursor cells belong to a transitory amplified population of cells derived from stem cells. Compared to stem cells, they have a limited capacity for self-renewal and differentiation. Such capacity for self-renewal (or proliferation) is demonstrated by the expression of proliferation markers such as, for example, Ki-67 nuclear antigen. Moreover, since progenitor cells are committed to a particular differentiation process, progenitor cells also express specific markers. Examples of progenitor cells that can be grown in an organoid of the present invention include, but are not limited to, hematopoietic progenitor cells, endothelial progenitor cells, neural progenitor cells, mesenchymal progenitor cells, osteogenic progenitor cells, stromal progenitor cells, and the like.

As used herein, the term **"*differentiated cells*"** refers to cells specialized for a particular function and that do not have the ability to generate other kinds of cells. Examples of adult differentiated cells that can be grown in an organoid of the present invention include, but are not limited to, basal cells, epithelial cells, platelets, lymphocytes, T-cells (T lymphocytes), B-cells (B lymphocytes), natural killer cells, reticulocytes, granulocytes, monocytes, mast cells, neurocytes, neuroblasts, glioblastoma, cytomegalic cells, dendritic cells, macrophages, blastomeres, endothelial cells, interstitial cells, Kupffer cells, Langerhans cells, littoral cells, tissue cells such as muscle cells and adipose cells, osteoblasts, fibroblasts, and the like.

Cells suitable for 3D culture in organoids according to the present invention may be primary cells (*i.e.*, cells isolated or harvested directly from a living tissue or organ), secondary cells *(i.e.,* cells derived from the culture and expansion of a cell obtained from a living tissue or organ) or immortalized cells (*i.e.*, established cell lines). Thus, cells from an established cell line (a cancer *(e.g.,* primary or metastatic) cell line or a non-cancer cell line) may be purchased from commercial resources (for example, from the American Type Culture Collection, Manassas, VA). Alternatively, the cells used to produce a multicellular organoid may be isolated or derived from *ex vivo* biological samples or obtained from volunteers or patients. Hence, the cells may be derived from any of a biopsy, a surgical specimen, an aspiration, a drainage, or a cell-containing fluid. Suitable cell-containing fluids include any of blood, lymph, sebaceous fluid, urine, cerebrospinal fluid, or peritoneal fluid. The cells may be obtained directly from the subject without intermediate steps of subculture, or they may first undergo an intermediate culturing step to produce a primary culture. Methods for harvesting cells from biological tissue and/or cell containing fluids are well known in the art. Generally, the cells are first dissociated or separated from each other before forming the cell suspension. Dissociation of cells may be accomplished by any conventional means known in the art, such as mechanically and/or chemically, *e.g.*, by treatment with enzymes. Preferably, the cells are treated to remove dead and/or dying cells and/or cell debris.

Cells to be used in the context of the present invention can be genetically engineered to express or produce a protein, nucleic acid, or another product of interest. Cells can be differentiated from reprogrammed cells or transdifferentiated from differentiated cells. Alternatively, or additionally, cells may be engineered to contain a gene of interest such as a gene expressing a growth factor or a receptor, or to contain a defective gene, or yet to contain Oct3/4, Sox2, Klf4, and c-Myc genes in order to prepare human induced stem cells from adult somatic cells.

### B. Cell Suspension and Introduction thereof inside a Liquid Pearl

An organoid is produced by introducing a cell suspension inside a liquid pearl. As used herein, the term **"*cell suspension*"** refers to a mixture of cells and a liquid medium, wherein the cells are suspended (*i.e.*, free-floating) in the liquid medium. As will be recognized by one skilled in the art, a cell suspension may contain any suitable concentration of cells. Generally, a suitable concentration of cells is one that is low enough to allow the cell suspension to be introduced (e.g., injected) into the liquid pearl without interfering with the introduction (e.g., without clogging the introduction system). A suitable concentration of cells is also one that is high enough to allow 3D cell culture inside an organoid. Depending on the nature of the cells used, one skilled in the art can easily determine the optimal cell concentration range to be used in the context of the present invention. In certain embodiments, the cells are suspended at a concentration range of 1×10² cells/mL of liquid medium to 1×10⁹ cells/mL of liquid medium, for example of 1×10³cells/mL to 5×10⁸ cells/mL or of 1×10⁴ cells/mL to 1×10⁷ cells/mL or yet of 1×10⁵ cells/mL to 5×10⁶ cells/mL. Methods of determining cell concentration are known in the art, for example, the cells may be counted with a hemocytometer.

The liquid medium in which cells are in suspension can be isotonic, hypotonic, or hypertonic. Generally, the liquid medium is aqueous. In certain embodiments, the liquid medium contains a buffer and/or at least one salt or a combination of salts (*e.g.*, sodium, potassium, magnesium, chloride, and/or acetate salts). The liquid medium is buffered so that it is maintained at physiologically pH. In some embodiments, the pH of the liquid medium ranges from about 5 to about 8,5, for example from about 6 to about 8, or from about 6.5 to about 7.5. A variety of pH buffers may be used to achieve the desired pH. Suitable buffers include, but are not limited to, Tris, MES, Bis-Tris, ADA, ACES, PIPES, MOPSO, Bis-Tris propane, BES, MOPS, TES, HEPES, DIPSO, MOBS, TAPSO, HEPPSO, POPSO, TEA, HEPPS, Tricine, Gly-Gly, Bicine, and a phosphate buffer (e.g., sodium phosphate or sodium-potassium phosphate, among others). The liquid medium may comprise from about 10 mM to about 100 mM buffer, about 25 mM to about 75 mM buffer, or from about 40 mM to about 60 mM buffer, among others. The type and amount of the buffer used in the liquid medium can vary from application to application. The liquid storage may contain human serum albumin (e.g., 0.1-10%).

Prior to use, the cell suspension may be centrifuged, for example at 200g for 5 minutes, to allow cell cluster formation and minimize aggregates formation.

Introduction of a small volume of cell suspension inside a liquid pearl may be carried out using any suitable method. In certain embodiments, the cell suspension is introduced by injection. The volume to be injected will generally be comprised between 2 µL and 30 µL, preferably between about 5 and about 10 µL, but may be about 15 µL or about 20 µL or about 25 µL.

It will be recognized by one skilled in the art that cells can be introduced into a liquid pearl by preparing the liquid pearl with a cell suspension in the solution of methylcellulose in a cell culture medium. However, in this case, the aggregation of the cells takes longer.

### C. Organoid Culture

Once the organoid is formed, 3D cell culture is conducted using appropriate culture conditions. As used herein, the term **"*culturing*"** refers to propagating or nurturing a cell or a population of cells, by incubation for a period of time in an environment and under conditions which support cell viability or propagation. Thus, the term **"*appropriate culture conditions*",** as used herein, refers to culture conditions that support survival and proliferation of cells cultured in an organoid. Such culture conditions are known in the art or may easily be determined and optimized by one skilled in the art. The culture conditions are the same as those used in 2D cultures, with the culture medium being adapted to the cultured cell phenotype.

An organoid is generally used immediately after preparation or following a step of culture.

### 2. Heterotypic or Co-culture Organoids

A particular advantage of the liquid pearls disclosed herein is that they allow the assembly of multiple, different cell types into multicellular aggregates (co-culture organoids) in a manner which closely resembles the natural formation of those multicellular aggregates *in vivo.* Thus, it is to be understood that an organoid according to the present invention may contain more than one cell phenotype, for example two different cell phenotypes or three different cell phenotypes, and that the determination of which cell phenotypes are used to generate the organoid will depend on its intended application. The present invention provides methods to generate heterotypic organoids. The terms **"*heterotypic organoid*"** and **"*co-culture organoid*"** are used herein interchangeably. They refer to an organoid that contains more than one cell phenotype.

In a first version, the method for generating a heterotypic organoid comprises steps of:
(1) producing a liquid pearl for 3D cell culture using a method as described herein or providing a liquid pearl produced using a method as described herein;
(2) introducing cells in suspension inside the liquid pearl to obtain a heterotypic organoid, wherein the cell suspension comprises more than one cell phenotype; and
(3) culturing the heterotypic organoid obtained.

In a second version, the method for generating a heterotypic organoid comprises steps of:
(1') generating a first organoid using a method as described herein or providing a first organoid generated using a method as described herein, wherein the first organoid comprises a first cell phenotype or cell composition;
(2') generating a second organoid using a method as described herein or providing a second organoid generated using a method as described herein, wherein the second organoid comprises a second cell phenotype or cell composition;
(3') placing the first organoid in close proximity to the second organoid to allow fusion of the first and second organoids into a heterotypic organoid,
wherein the first cell phenotype or cell composition and the second cell phenotype or cell composition are different.

This method may further comprise steps of:
(4') generating a third organoid using a method as described herein or providing a third organoid generated using a method as described herein, wherein the third organoid comprises a third cell phenotype or cell composition;
(5') placing the third organoid in close proximity to the heterotypic organoid obtained in step (3') to allow fusion of the third and heterotypic organoids into a larger heterotypic organoid.

### A. Cell Phenotypes and Cell Compositions

A heterotypic organoid is generated by fusion of at least two organoids, the at least two organoids comprising different cell phenotypes or cell compositions. As used herein, the term **"*cell phenotype*"** refers to a classification used to identify cells that share morphological or phenotypical features. Cells may be of the same genotype, but of different cell type due to the differential regulation of the genes that contain. Examples of cell phenotypes include, but are not limited to, beta cells, neuronal cells, cardiomyocyte cells, megakaryocyte cells, endothelial cells, epithelial cells, red blood cells, lymphocytes, monocytes, macrophages, granulocytes, hepatocytes, nephrogenic cells, adipogenic cells, osteoblast cells, osteoclastic cells, alveolar cells, cardiac cells, intestinal cells, renal cells, retinal cells, melanocytes, keratinocytes, mesenchymal stem cells, adipose stem cells, embryonic stem cells, umbilical cord blood derived stem cells, smooth muscle cells, skeletal myoblasts, and the like. For example, with the goal of developing a heterotypic organoid that mimics the human heart microenvironment, a first organoid may be prepared using cardiac myocytes (first cell type) and a second organoid may be prepared using cardiac fibroblasts (second cell type). Alternatively, the first organoid may be prepared using human induced pluripotent stem cell-derived cardiomyocytes (first cell phenotype) and the second organoid may be prepared using cardiac fibroblasts (second cell phenotype).

As used herein, the term **"*cell composition*"** refers to a plurality of cells of different phenotypes. For example, a cell composition may be derived from a biological tissue specimen and therefore contains a mixture of the cell phenotypes present in the tissue. Alternatively, a cell composition may result from the mixture of at least two cell populations of different phenotypes. Thus, a heterotypic organoid prepared using a method of fusion described herein comprises a cell composition, *i.e.*, a mixture of at least two different cell phenotypes. For example, a heterotypic cardiac organoid may be obtained by fusion of a first organoid comprising a cell composition of human induced pluripotent stem cell-derived cardiomyocytes (first cell phenotype) and cardiac fibroblasts (second cell phenotype) and a second organoid prepared using human coronary artery endothelial cells (third cell phenotype).

It will be recognized by one skilled in the art that the different cell phenotypes used in the generation of a heterotypic organoid will depend on the intended use of the organoid resulting from the fusion(s). Preferably, the cells of the different cell phenotypes are cultured at ratios approximating those present *in vivo* in order to better mimic the natural environment. Such ratios are known to those skilled in the art or may easily be determined.

### B. Fusion of Organoids

Fusion of two organoids to form a co-culture organoid or larger organoid is initiated by placing a first organoid in close proximity to a second organoid, or more specifically by placing a first liquid pearl comprising a first organoid in close proximity to a second liquid pearl comprising a second organoid.

As used herein in connection with two organoids, the term **"*fusion*"** refers to a process in which neighboring liquid pearls, each comprising an organoid, merge or coalesce to form a larger liquid pearl that encompasses the content of the first and the second organoids. The volume of the resulting organoid is higher than the volume of the first organoid or of the second organoid. In general, an organoid obtained by fusion of two liquid pearls has a volume of between 100 µL and 700 µL, for example between 200 µL and 600 µL, or between 300 µL and 500 µL, or between 350 µL and 450 µL.

As used herein, the term **"*close proximity*"** refers to a distance that allows fusion between the two liquid pearls. One skilled in the art knows how to determine such a distance. In certain embodiments, close proximity means side-by-side or adjacent *(i.e.,* a configuration in which at least a portion of the external surface of the first liquid pearl is in direct contact with at least a portion of the external surface of the second liquid pearl). In other embodiments, close proximity means a separation distance between the external surface of the first liquid pearl and the external surface of the second liquid pearl of less than 50 µm, for example about 10 µm, about 20 µm, about 30 µm, about 40 µm or about 50 µm. In certain embodiments, close proximity means presence of the first liquid pearl and the second liquid pearl in the same well of a multiwell plate.

Two liquid pearls may be placed in close proximity using any suitable method known in the art. For example, a first liquid pearl may be picked up with a capillary tube attached to a microdispenser or micromanipulator and transferred to the well of a multiwell-plate, the well containing a second liquid pearl. The pair of liquid pearls may then be allowed to fuse over a time period sufficient for fusion, for examples for a few seconds, or a few minutes (e.g., 1 minutes, 2 minutes, 5 minutes, 10 minutes, 15 minutes, 30 minutes, 45 minutes), or for over 1 hour, over 3 hours, over 6 hours, over 12 hours, over 24 hours or more.

### 3. Cancerous Organoids or Patient-Derived Organoids

The cell communication process and its regulation are of major importance in the cancer contest, where cancer cells interact and communicate with a variety of non-cancerous cells, including normal epithelial cells, immune cells, endothelial cells, and fibroblasts, which collectively constitute the tumor microenvironment (TME). 3D co-culture systems, such as organoids, offer a promising tool to model the interactions between cancer cells and the other cells that compose the TME. Thus, a method according to the present invention may be used to generate a cancerous or cancer-like organoid or a patient-derived organoid (a term that is more specifically used when the cancer cells are derived from a tumor tissue or fluid of a patient).

### A. Cancer Cells

A cancerous organoid or patient-derived organoid comprises cancer cells. As used herein, the term **"*cancer cell*"** refers to a cell or immortalized cell line derived from a tumor or cancer. The term "*cancer cell*" may also refer to a cell that exhibits cancer-like properties, e.g., uncontrollable reproduction, self-sufficiency in growth signals, insensitivity to anti-growth signals, ability to metastasize, loss of ability to undergo programmed cell death *(e.g.,* apoptosis), and/or sustained angiogenesis. The term "*cancer cells*" is meant to encompass pre-malignant, malignant, pre-metastatic, metastatic, and non-metastatic cells.

In certain embodiments, the cancer cells used in the preparation of a cancer-like organoid are from a solid tumor. Thus, the cancer cells may be mesothelioma cells, melanoma cells, adenoma cells, carcinoma cells, adenocarcinoma cells, ductal carcinoma cells, rhabdomyosarcoma cells, osteosarcoma cells, neuroblastoma cells, astrocytoma cells, or glioblastoma cells. Carcinoma cells may be from an adenocarcinoma, an adrenocortical carcinoma, a colon adenocarcinoma, a colorectal adenocarcinoma, a colorectal carcinoma, a ductal cell carcinoma, a lung carcinoma, a thyroid carcinoma, a hepatocellular carcinoma, a nasopharyngeal carcinoma, or an unspecified carcinoma. The cancer cells may be from a melanoma *(e.g.,* a malignant melanoma) or a non-melanoma skin carcinoma. The cancer cells may be from a desmoid tumor, a desmoplastic small round cell tumor; an endocrine tumor, an Ewing sarcoma, a germ cell tumor (*e.g.*, testicular cancer, ovarian cancer, choriocarcinoma, endodermal sinus tumor, germinoma, etc.), a hepatoblastoma, a neuroblastoma; a non-rhabdomyosarcoma soft tissue sarcoma; an osteosarcoma, a retinoblastoma, a rhabdomyosarcoma, or a Wilms tumor. The cancer cells may be from an acoustic neuroma; an astrocytoma (*e.g.*, a grade I pilocytic astrocytoma, a grade II low-grade astrocytoma, a grade III anaplastic astrocytoma, or a grade IV glioblastoma multiforme); a chordoma; a craniopharyngioma; a glioma *(e.g.,* a brain stem glioma; an ependymoma; a mixed glioma; an optic nerve glioma; or a subependymoma); a glioblastoma; a medulloblastoma; a meningioma; a metastatic brain tumor; an oligodendroglioma; a pineoblastoma; a pituitary tumor; a primitive neuroectodermal tumor; or a schwannoma. The cancer cells may be from pancreatic cancer, breast cancer, brain cancer, renal cancer, prostate, cervical cancer, liver cancer, colorectal cancer, ovarian cancer, colon cancer, testicular cancer, thyroid cancer, lung cancer, or breast cancer. In certain embodiments, the cancer cells from these types of solid cancers/tumors are from cell lines.

In certain embodiments, the cancer cells are from a non-solid tumor. The cancer cells may be leukemia cells, acute myelogenous leukemia cells, acute myeloid leukemia cells, acute T cell leukemia cells, acute lymphoblastic leukemia cells, hairy cell leukemia cells, acute promyelocytic leukemia cells, lymphoma cells, Burkitt's lymphoma cells, B cell chronic lymphocytic leukemia cells, non-Hodgkin's lymphoma cells, Hodgkin's lymphoma cells, or multiple myeloma cells. In certain embodiments, the cancer cells are tumor stem cells or cancer stem cells. In certain embodiments, the cancer cells from these types of non-solid cancers are cell lines.

Cancer cell lines are known in the art. Examples of cancer cell lines include, but are not limited, carcinoma cell lines (such as 5637, BT-20, BT-474, CAMA-1, HCC2218, SW527, MDA-MB-453, MDA-MB-4355, T-47D, ZR-75-1, UACC-812, HCC1419, RKO, LS411N, T84, KATO III, NCI-N87, SNU-16, A-498, Caki-1, Bel-7402, Bel-7404, HEP-3B, HepG2, A-427, A549, SW1573, NCI-H358, NCI-H460, NCI-H292, NCI-H82, NCI-H226, NCI-H526); osteosarcoma cell lines (such as KHOS/NP, MNNG/HOS, Saos-2, U-2 OS, SJSA-1); astrocytoma cell lines (such as CCF-STTG1); glioblastoma cell lines (such as DBTRG-05MG, U87 MG, T98G, ); neuroblastoma cell lines (such as SK-N-SH, SK-N-AS); adenocarcinoma cell lines (such as MCF-7, MDA-MB-231, MDA-MB-436, SK-BR-3, HeLa, Caco-2, COL0205, COL0320/DM, DLD-1, HCT-15, SK-CO-1, SW48, SW480, HCT-8, AGS, 769-P, 786-0, ACHN, SK-HEP-1, Calu-3, NCI-H1395, NCI-H1975, SK-LU-1, NCI-H2122); Leiomyoblastoma cell lines (such as G-402); leukemia cell lines (such as CML-T1, CTV-1, JVM-2, K562, MHH-CALL2, NALM-6, 8E5, CCRF-SB, CEM/C1, CEM/C2, CEM-CM3, CCRF-HSB-2, KG-1, KG-1a, CCRF-CEM, MOLT-3, SUP-B15, TALL-104, Loucy, RS4; 11, REH, AML-193, THP-1, MOLM-13, Kasumi-1, Kasumi-3, BDCM, HL-60, I 2.1, I 9.2, J.gamma1.WT, J.RT3-T3.5, P116, P116.c139 [P116.c39], D1.1, J45.01, MV-4-11, Kasumi-4, MEG-01, KU812, Mo, JM1, GDM-1, CESS, ARH-77); and mesothelioma cell lines (such as MSTO-211H).

In certain embodiments, the cancer cells are obtained from a patient's tumor. Techniques known to one skilled in the art may be used to obtain cancer cells from a patient's tumor. Cancer cells may be derived from any one of a surgical specimen, an aspiration, a drainage, or a cell-containing fluid *(e.g.,* blood, lymph, sebaceous fluid, urine, cerebrospinal fluid, or peritoneal fluid). In some embodiments, a biopsy of a patient's tumor is obtained. The biopsy can be from any organ or tissue, for example, skin, liver, lung, heart, colon, kidney, bone marrow, teeth, lymph node, hair, spleen, brain, breast, or other organs. Any biopsy technique known to those skilled in the art can be used for isolating a tumor sample from a patient, for instance, open biopsy, close biopsy, core biopsy, incisional biopsy, excisional biopsy, or fine needle aspiration biopsy. Before the cancer cells are isolated, the tumor biopsy may be stored for a period of time ranging from 30 minutes to several hours (1 hour to 24 hours), to several days (2 days to 7 days), to several weeks (1 week to three weeks) to several months (1 month to 3 months or more). Alternatively, cancer cells are obtained immediately after the tumor biopsy has been obtained from a patient.

In certain embodiments, the cancer cells used in the preparation of cancer-like organoids consist of, or comprise, cancer stem cells. The term **"*cancer stem cell*"** refers to a sub-population of cancer cells found within tumor or hematological cancers that possess characteristics normally associated with stem cells. These cells are tumorigenic (tumor-forming), in contrast to the bulk cancer cells, which are non-tumorigenic. There is mounting evidence that such cells exist in almost all tumor types. Cancer stem cells have stem cell properties such as self-renewal and the ability to differentiate into multiple cell types. They persist in tumors as a distinct population, and they give rise to the differentiated cells that form the bulk of the tumor mass and phenotypically characterize the disease. Cancer stem cells have been demonstrated to be fundamentally responsible for carcinogenesis, cancer metastasis, and cancer reoccurrence. Cancer stem cells are also often called tumor initiating cells, cancer stem-like cells, stem-like cancer cells, highly tumorigenic cells, or super malignant cells. Methods of identification and isolation of cancer stem cells have been reported. As known in the art, cancer stem cells may be used to generate organoids known as tumorspheres (*i.e.*, models of cancer stem cell culture and expansion). Examples of tumorspheres include, but are not limited to, mammospheres, neurospheres, cardiospheres, chondrospheres, osteospheres, gliomaspheres, hepatospheres and the like.

### B. Other Cells

The interactions between normal and cancer cells, in the tumor microenvironment, can be tumor-suppressive, *i*.*e*., induce an immunoreaction against cancer, but often eventually become tumor-promoting. Overall, interactions within the tumor microenvironment promote cancer progression. Therefore, understanding cell-cell interactions with the tumor microenvironment or mimicking cell-cell interactions of the *in vivo* tumor microenvironment will not only provide insight into cancer biology but also the potential for identifying new therapeutics. Thus, in addition to cancer cells, a cancer-like organoid generated according to a method of the present invention may further comprise immune cells, endothelial cells, epithelial cells, fibroblasts, and any combination thereof.

As used herein, the term **"*immune cells*"** refers to cells of hematopoietic origin that are involved in the specific recognition of antigens. Immune cells include neutrophils, eosinophils, basophils, mast cells, monocytes, macrophages, dendritic cells, natural killer cells, and lymphocytes (B cells and T cells). Immune cells have antithetic functions in the tumor microenvironment (TME), having both pro- and anti-tumor activity. Immune cells used in the context of the present invention may be primary cells (obtained from a subject), secondary cells or cells from an established cell line, as indicated above. Immune cells used for preparing a cancer-like organoid may be activated or non. Methods of immune cells activation are known in the art.

*In vivo*, cancer cells interact with endothelial cells to elongate blood vessels in the tumor microenvironment. As used herein, the term **"*endothelial cells*"** refers to cells that line the interior surface of blood vessels and lymphatic vessels. In certain preferred embodiments, the endothelial cells used in the context of the present invention are vascular endothelial cells. Endothelial cells used for preparing a cancer-like organoid may be primary cells (obtained from a subject), secondary cells or cells from an established cell line, as indicated above. For example, human umbilical vein endothelial cells (HUVEC) may be used. HUVEC are primary cells isolated from the vein of the umbilical cord. They are a model system for studying endothelial cell function, with applications including hypoxia, inflammation, oxidative stress, response to infection, and both normal and tumor-associated angiogenesis.

The effect of normal cells on cancer cells provides a fascinating mechanism for self-defense against cancer. For example, when a single epithelial cell is transformed by an oncogene and is surrounded by normal epithelial cells, the transformed cell is, in many cases, apically extruded and thus eliminated from the epithelium (Hogan et al., Int. J. Biochem. Cell Biol., 2011, 43: 496-503; Kajita et al., J. Cell Sci., 2010, 123: 171-180). In another example, normal breast epithelial cells or their conditioned media attenuate the growth of co-culture breast cancer cells (Dong-Le Bourhis et al., J. Int. J. Cancer, 1997, 71: 42-48; Spink et al., Cell Biol. Int., 2006, 30: 227-238.). As used herein, the term **"*epithelial cells*"** refers to cells of the epithelium, a thin, continuous, protective layer of compactly packed cells with little intercellular matrix. Epithelial tissues line the outer surfaces of organs and blood vessels throughout the body, as well as the inner surfaces of cavities in many internal organs. Epithelial cells used for preparing a cancer-like organoid may be primary cells (obtained from a subject), secondary cells or cells from an established cell line, as indicated above.

Contrary to the preventive effect of the microenvironment, once cancer cells override the self-defense mechanisms and begin to form a tumor, the surrounding cells such as fibroblasts acquire the ability to aid further growth of cancer cells (Kalluri et al., Nat. Rev. Cancer, 2006, 6: 392-401; Bhowmick et al., Nature, 2004, 432: 332-337) Fibroblasts from breast carcinomas promote the growth of cancer cells more significantly than normal mammary fibroblasts, suggesting co-evolution of cancer and surrounding fibroblasts (Orimo et al., Cell, 2005, 121: 335-348). This supportive mechanism is seen both in primary locations and in metastatic regions (Mathot et al., Cancer Sci., 2012, 103: 626-631). As used herein, the term "***fibroblast***" refers to a specific type of cell found in connective tissue (skin, tendons, and other tough tissues). Fibroblasts synthesize the extracellular matrix and collagen, produce the structural framework (stroma) for animal tissues, and play an important role in healing wounds. Fibroblasts used for preparing a cancer-like organoid may be normal fibroblasts or cancer-associated fibroblasts. They may be primary cells (obtained from a subject), secondary cells or cells from an established cell line, as indicated above.

In a particular embodiment, a heterotypic cancer-like organoid generated using a method according to the present invention comprises three types of cells, wherein cells of the first phenotype are cancer cells, cells of the second phenotype are endothelial cells, in particular vascular endothelial cells, and cells of the third phenotype are immune cells, in particular natural killer cells.

It will be recognized by one skilled in the art that the different cell phenotypes used in the generation of a cancer-like organoid will depend on the intended use of the organoid resulting from the fusion(s). Preferably, the cells of the different phenotypes are cultured at ratios approximating those present *in vivo* in order to better mimic the natural environment of the tumor. Such ratios are known to those skilled in the art or may easily be determined.

The heterotypic organoids, including the cancer-like organoids, formed using a method of the present invention, may be characterized in that they exhibit characteristics that substantially mimic those of the tissue of origin (*e.g.*, tumor in the case of a cancer-like organoid). Thus, at least one of the antigen profile, genetic profile, tumor biology, tumor architecture, gas concentrations, cytokine expression, growth factor expression and cell adhesion profile of one or more multicellular organoids may be substantially identical to that of the tissue of origin. Accordingly, the heterotypic organoids, including the cancer-like organoids, exhibit a substantially similar or identical behavior to that of natural cell systems, for example with respect to organization, growth, viability, cell survival, cell death, metabolic and mitochondrial status, oxidative stress and radiation response as well as drug response.

### III - Uses and Applications of Organoids

Multicellular organoids prepared according to a method described herein may be used in an array of applications for research, diagnostic and/or therapeutic purposes, including, but not limited to, for examining cell interactions (for example for cell transplantation therapies or drug testing purposes), biomarker identification, tumor profiling, drug screening (*e.g.*, candidate compound identification and/or testing, pharmacokinetic profiling, pharmacodynamic profiling, efficacy studies, cytotoxicity studies, penetration studies of compounds, therapeutic resistance studies), personalized or tailored therapies, and the like.

### 1. Cell-Cell Interactions, Drug Screening and Development

In particular, the present invention relates to a method of assessing the effect of an agent on a property of a cell in an organoid, the method comprising steps of:
(I) producing an organoid using a method as described herein or providing an organoid prepared using a method as described herein, wherein the cell is in contact with the agent inside the organoid; and
(II) assessing the effect of the agent on the property of a cell in the organoid.

The cell may be brought into contact with the agent using any of a variety of methods. Thus, the agent may be applied to *(e.g.,* by injection into) the organoid following preparation and optionally culture thereof. Alternatively, the agent may be added to the solution of methylcellulose in a cell culture medium that is used for the formation of the liquid pearl (see step (b)). Alternatively, the cell suspension used to generate the organoid may be contacted with the agent prior to being introduced inside the liquid pearl (see step (2)). Alternatively, the agent may be brought into contact with the cell by fusion of two liquid pearls, the first liquid pearl containing the organoid and the second liquid pearl containing the agent (see step (3')). Depending on the nature of the agent and the effect that is intended to be assessed, one skilled in the art will be able to select the most appropriate contacting method.

The property of a cell (or cells or cell population) that may be assessed using an organoid according to the present invention may be selected from the group consisting of survival, growth, proliferation, differentiation, migration, morphology, signalling, metabolic activity, gene expression, cell-cell interaction, and any combination thereof. Assessing one or more properties of a cell may be carried out using any suitable method known in the art, either from cell organoids or from organoids fixed by snap-freezing or chemical fixation techniques. For example, any cell survival, growth, proliferation, differentiation, migration, and morphology may be assessed by microscopy or image analysis. Properties may be detected using appropriate markers. For example, expression of detectably-labelled proteins, reporters and/or single-step labelling of cell components and markers can enable cell architecture, multicellular organization, and other readouts to be directly visualized, for example by fluorescence microscopy. Gene expression may be assessed by functional genomic (*e.g.*, microarray) techniques, and so on. It is appreciated that one skilled in the art may selected the appropriate technique to assess a given property.

It will be appreciated that the method allows the assessment of how a particular cell phenotype affects the function of another cell phenotype, for example where different cell phenotypes are present in the same organoid. Thus, in certain embodiments, the agent is a further cell or cell composition and the effect of the further cell or cell composition is assessed. For example, the method of the present invention may be used to study cell-cell interactions in the complex tissue microenvironment, and identify the resulting effect on tumor progression and metastasis.

As will be recognized by those of ordinary skill in the art, any kind of agents can be tested using a method described herein. An agent may be a synthetic or natural compound; it may be a single molecule, or a mixture or complex of different molecules. It may belong to any of a large variety of classes of chemicals, including proteins, peptides, peptidomimetics, peptoids, polypeptides, saccharides, steroids, RNA agents *(e.g.,* mRNA or siRNA), antibodies, ribozymes, antisense oligonucleotides, small molecules, and the like. The term **"*small molecule*",** as used herein, refers to any natural or synthetic organic or inorganic compound or factor with a low molecular weight. Preferred small molecules have molecular weights of more than 50 Daltons and less than 2,500 Daltons. More preferably, small molecules have molecular weights of less than 600-700 Daltons. Even more preferably, small molecules have molecular weights of less than 350 Daltons.

The agent may be a drug or a biologically active agent. The agent may be an inhibitor of a particular cellular function. It will be appreciated that the method allows one to assess the function of a cellular gene or protein, for example by using an agent that is an inhibitor of that gene or protein. For instance, the agent may be any of a chemical inhibitor, a peptide inhibitor, a siRNA molecule or a shRNA construct or any agent capable of effecting a gene knockdown. It may also be desirable to use more than one inhibitor (*e.g.*, with different selectivities) to provide further insight into the function of a cellular gene or protein. Similarly, by exposing multiple organoids to a range of respective agents *(e.g.,* RNAi inhibitors), the methodology can be scaled up to investigate the cellular functions of genome or proteome arrays on a larger scale.

It is appreciated that an organoid disclosed herein constitutes a robust, suitable tool for drug discovery. Thus, the method includes identifying an agent that modulates one or more properties of a cell as a potential therapeutic agent.

In certain embodiments, a method may be used for testing a single agent or a few agents. In other embodiments, the method is used for screening collections or libraries of agents *(i.e.,* candidate compounds). As used herein, the term **"*collection*"** refers to any set of compounds, molecules, or agents, while the term **"*library*"** refers to any set of compounds, molecules or agents that are structural analogs.

Collections of natural compounds in the form of bacterial, fungal, plant and animal extracts are available from, for example, Pan Laboratories (Bothell, WA) or MycoSearch (Durham, NC). Libraries of candidate compounds that can be screened using the methods of the present invention may be either prepared or purchased from a number of companies. Synthetic compound libraries are commercially available from, for example, Comgenex (Princeton, NJ), Brandon Associates (Merrimack, NH), Microsource (New Milford, CT), and Aldrich (Milwaukee, WI). Libraries of candidate compounds have also been developed by and are commercially available from large chemical companies, including, for example, Merck, Glaxo Welcome, Bristol-Meyers-Squibb, Novartis, Monsanto/Searle, and Pharmacia UpJohn. Additionally, natural collections, synthetically produced libraries and compounds are readily modified through conventional chemical, physical, and biochemical means. Chemical libraries can be prepared by traditional or automated synthesis, or proprietary synthetic methods (see, for example, DeWitt et al., Proc. Natl. Acad. Sci. U.S.A. 1993, 90:6909-6913; Zuckermann et al., J. Med. Chem. 1994, 37: 2678-2685; Carell et al., Angew. Chem. Int. Ed. Engl. 1994, 33: 2059-2060; Myers, Curr. Opin. Biotechnol. 1997, 8: 701-707). Candidate compounds may also be obtained by any other of the numerous approaches in combinatorial library methods known in the art, including peptoid libraries, spatially addressable parallel solid phase or solution phase libraries; synthetic library methods requiring deconvolution; the "one-bead one-compound" library method; and synthetic library methods using affinity chromatography selection.

In one particular embodiment, the agent is a drug-like compound or a lead compound for the development of a drug-like compound. The term **"*drug-like compound*"** is well known to those skilled in the art, and refers a compound that has characteristics that may make it suitable for use in medicine, for example as the active ingredient of a medicament. A drug-like compound may additionally exhibit features of selective interaction with a particular protein or proteins and be available and/or able to penetrate target cellular membranes or the blood/brain barrier, but it will be appreciated that these features are not essential. The term **"*lead compound*"** is similarly well-known to those skilled in the art, and refers to a compound, whilst not itself suitable for use as a drug (for example because it is only weakly potent against its intended target, non-selective in its action, unstable, poorly soluble, difficult to synthesize or has poor bioavailability) may provide a starting point for the design of other compounds that may have more desirable characteristics. Thus, in one embodiment, the method further comprises modifying an agent which has been shown to modulate at least one of the properties listed above, and testing the ability of the modified agent to modulate at least one of the properties listed above.

It is appreciated that assays which are capable of high throughput operation are particularly preferred. Thus, the assays may be performed on multiple organoids contained in multi-well plates. In certain embodiments, the assays are automated or semi-automated. Thus, in one embodiment, the assessment of the one or more of the cell properties listed above is automated.

In certain preferred embodiments, the cell (or cells or cell population) is located in a cancer-like organoid, as described above. Thus, the method may be used to assess various properties of cancer cells or to determine the effects of particular agents (*e.g.*, candidate drugs) on, for example, cancer cell invasion or migration. However, it is appreciated that the properties of other cell phenotypes may also be assessed including stem cells, endothelial cells, and immune cells and that the methods have equal application in any one or more of stem cell biology, angiogenesis, immune biology, toxicity studies and tissue engineering. For example, it is within the scope of the invention, to rebuild a metastatic microtumor, e.g., tumor cells with hepatocytes, or tumor cells with bone marrow cells.

Typically, in a screening method, cells are exposed to the agent at a concentration ranging from about 1 fM to about 10 mM. Preferably, the concentrations used are between about 10 pM and about 100 µM. It is of course possible to test other concentrations without deviating from the present invention. Each agent can, moreover, be tested, in parallel, at different concentrations. Incubation can be maintained for any suitable length of time, for example between a few minutes and several hours or days, particularly between 5 and 72 hours, generally between 12 and 48 hours. In one embodiment, the agent may be contained within a bead so as to control the rate at which the agent is released *(e.g.,* slow release).

### 2. Personalized Medicine

Cancer heterogeneity causes variability in treatment response among patients. Precision oncology has emerged to address this problem and focuses on creating an individualized treatment plan by identifying prognostic markers and therapeutics. Personalized medicine obtains information about the disease of a specific patient to select a drug and the correct dosage, as well as to optimize treatment procedure. Tumor-like organoids, which recapitulate genomic and histological features of the original tumor from which they were prepared, can hence be utilized for personalized cancer medicine (Sachs et al., Cell, 2018, 172: 373-386; Gao et al., Cell, 2014, 159: 176-187; Kopper et al., Nat. Med., 2019, 25: 838-849; Tiriac et al., Cancer Discov., 2018, 8: 1112-1129; Fujii et al., Cell Stem Cell, 2016, 18: 827-838; Driehuis et al., Cancer Discov., 2019, 9: 852-871). Therefore tumor-like organoids, prepared from the tumor of a patient, have tremendous potential for screening anti-cancer drugs, optimizing immunotherapy and identifying prognostic biomarkers (Montazeri et al., Trends Biotechnol., 2018, 36: 358-371). Thus, in addition to being useful in drug screening and development, organoids, prepared using a method described herein from the tumor of a patient, may also be useful in personalized medicine. For example, a tumor-like organoid may be used to test the safety or efficacy of the potential drug treatment, and so may aid the customization (or personalization) of treatment of individual patients.

The invention will be further illustrated by the following figures and examples. However, these examples and figures should not be interpreted in any way as limiting the scope of the present invention.

### Examples

The following examples describe some of the preferred modes of making and practicing the present invention. However, it should be understood that the examples are for illustrative purposes only and are not meant to limit the scope of the invention. Furthermore, unless the description in an Example is presented in the past tense, the text, like the rest of the specification, is not intended to suggest that experiments were actually carried out or data were actually obtained.

### Examples 1: Cannabis Sativa Extract Induces Apoptosis in Human Pancreatic 3D Cancer Models: Importance of Major Antioxydant Molecules Present Therein

### Materials and Methods

In a first step, medium filling the pearls is prepared using DMEM^{high} glucose complete medium supplemented by 1.2 % of methylcellulose solution (MC) at a ratio of 4 to 1. For spheroid generation, 150 µLof pearls medium were dispensed onto fumed silica-coated 6-well plates, then Aspc-1 cells, at a density of 10,000 cells/mL, were directly injected into each pearl. The injected pearls were then incubated in a humidified incubator at 37C° and 5% CO₂.

After 24 hours, 50 µL of complete medium containing the different extracts (final concentration 80 µg/mL) were injected into each pearl and incubated for an additional 48 hours. To evaluate cell viability, pearls were transferred into 96-well plates and incubated for 30 minutes avec injection of Calcein AM (Thermofisher, France) and propidium iodide (Life technology, Fischer scientific) according to the manufacturer instructions. Pearls containing cancerous organoids were finally analyzed on Celigo image cytometer (Cyntellect Inc, San Diego, CA, USA).

### Results

### Pancreatic Cancerous Cells Responses to # 138-10D Extract Treatments

Prior to mechanistic investigations, the first step of the present study was to estimate the potential of the # 138-10D extracts in a model closest to the xerograph nude mouse model, as human pancreatic cancers cells are cultivated as cancerous 3D organoids in liquid pearls. To compare such activity to a drug used in human anti-cancer therapy, Gemcitabine occured to be the best candidate, as it is largely used in patients with pancreatic cancers.

The results obtained are presented on Figure 4.

### Conclusions

Pancreatic cancerous organoids, generated for 24 hours in liquid pearls, were found to respond to # 138-10D extract treatment, after 48 hours incubation, in a manner equivalent to 80 µM Gemcitabine used in therapy as a first-line treatment alone for pancreatic cancer (Figure 4). Comparable results were observed with a concentration of 80 µg/mL of # 138-10D extract, which represents an equivalent concentration of 40 µM CBD. It is interesting to note that Gemcitabine, one of the drugs, which is a marketed drug widely used in patients with pancreatic cancers, was only active on AsPC-1 cells at a concentration of 80 µM in our experimental conditions.

## Claims

1. A method for generating an organoid, the method comprising steps of:
(1) producing or providing a liquid pearl for 3D cell culture;
(2) introducing cells in suspension inside the liquid pearl to obtain an organoid; and
(3) culturing the organoid obtained,
wherein, the liquid pearl for 3D cell culture is produced by a method comprising steps of:
(a) coating a surface of a Petri dish with hydrophobic fumed silica;
(b) pipetting a volume v of a solution of methylcellulose in a cell culture medium and placing it on the coated surface of the Petri dish; and
(c) applying a gentle orbital shake to the Petri dish to form a liquid pearl.

2. The method for generating an organoid according to claim 1, wherein the method used for producing the liquid pearl for 3D cell culture further comprises a step of:
(d) pipetting the liquid pearl formed in step (c) and placing it in a well of a U-bottom multiwell-plate.

3. The method for generating an organoid according to claim 1 or claim 2, wherein the solution of methylcellulose in a cell culture medium comprises between 0.1 and 1.5% (w/v) of methylcellulose, in particular between 0.1 and 0.7% (w/v) of methylcellulose, preferably 0.3% (w/v) of methylcellulose, and/or wherein the cell culture medium is Dulbeccos' Modified Eagles Medium (DMEM).

4. The method for generating an organoid according to any one of claims 1 to 3, wherein, in step (b), the volume v of the solution of methylcellulose in a cell culture medium is comprised between 50 µL and 250 µL.

5. The method for generating an organoid according to any one of claims 1 to 4, wherein in the cell suspension of step (b), the cells are suspended at a concentration range of 1×10² cells/mL to 1×10⁹ cells/mL, in particular of 1×10³ cells/mL to 5×10⁸ cells/mL or of 1×10⁴ cells/mL to 1×10⁷ cells/mL or of 1×10⁵ cells/mL to 5×10⁶ cells/mL.

6. The method for generating an organoid according to any one of claims 1 to 5, wherein the cells in suspension are stem cells, progenitor cells or differentiated cells of human or mammal origin.

7. The method for generating an organoid according to claim 6, wherein the cells in suspension are primary cells, secondary cells or immortalized cells originating from healthy or diseased biological tissue or fluid.

8. The method for generating an organoid according to claim 7, wherein the cells in suspension are derived from a patient suffering from a disease, in particular a cancer patient.

9. The method for generating an organoid according to any one of claims 1 to 8, wherein in step (2), the volume of cell suspension introduced inside the liquid pearl is comprised between 2 µL and 30 µL.

10. A method for generating a heterotypic organoid, the method comprising steps of:
(1') generating a first organoid inside a first liquid pearl using a method according to any one of claims 1 to 9 or providing a first organoid inside a first liquid pearl generated using a method according to any one of claims 1 to 9, wherein the first organoid comprises a first cell phenotype or cell composition;
(2') generating a second organoid inside a second liquid pearl using a method according to any one of claims 1 to 9 or providing a second organoid inside a second liquid pearl generated using a method according to any one of claims 1 to 9, wherein the second organoid comprises a second cell phenotype or cell composition;
(3') placing the first liquid pearl in close proximity to the second liquid pearl to allow fusion of the first and second liquid pearls into a fusion liquid pearl comprising a heterotypic organoid,
wherein the first cell phenotype or cell composition and the second cell phenotype or cell composition are different.

11. The method for generating a heterotypic organoid according to claim 10, wherein the method further comprises steps of:
(4') generating a third organoid inside a third liquid pearl using a method according to any one of claims 1 to 9 or providing a third organoid inside a third liquid pearl generated using a method according to any one of claims 1 to 9, wherein the third organoid comprises a third cell phenotype or cell composition;
(5') placing the third liquid pearl in close proximity to the fusion liquid pearl comprising the heterotypic organoid obtained in step (3') to allow fusion of the third liquid pearl and the fusion liquid pearl into a larger fusion liquid pearl comprising a heterotypic organoid.

12. The method for generating a heterotypic organoid according to claim 10, wherein the method is used for generating a cancer-like organoid, cells of the first cell phenotype are cancer cells, and cells of the second cell phenotype are selected from the group consisting of epithelial cells, immune cells, endothelial cells, and fibroblasts.

13. The method for generating a heterotypic organoid according to claim 11, wherein the method is used for generating a cancer-like organoid, cells of the first cell phenotype are cancer cells, cells of the second cell phenotype are endothelial cells, in particular vascular endothelial cells, and cells of the third cell phenotype are immune cells, in particular natural killer cells.

14. A method of assessing the effect of an agent on a property of a cell in an organoid, the method comprising steps of:
(I) generating an organoid using a method according to any one of claims 1 to 9 or a method according to any one of claims 10 to 13 or providing an organoid generated using a method according to any one of claims 1 to 9 or a method according to any one of claims 10 to 13, wherein the cell is in contact with the agent inside the organoid;
(II) assessing the effect of the agent on the property of the cell in the organoid.

15. The method of assessing the effect of an agent on a property of a cell in an organoid according to claim 14, wherein the property of the cell is selected from the group consisting of survival, growth, proliferation, differentiation, migration, morphology, signalling, metabolic activity, gene expression, cell-cell interaction, and any combination thereof.

16. The method of assessing the effect of an agent on a property of a cell in an organoid according to claim 14 or claim 15, wherein the agent is a cell of a different cell phenotype or is a synthetic or natural compound or molecule.

17. The method of assessing the effect of an agent on a property of a cell in an organoid according to any one of claims 14 to 16, wherein the method is high throughput and/or automated.
